# EUROPEAN PATENT APPLICATION

(11) **EP 2 604 291 A1**
(43) Date of publication of application: **19.06.2013**
(21) Application number: 12194925.9
(22) Date of filing: 29.11.2012
(51) Int. Cl.: A61L 2/04, A61L 2/06, A61L 2/07, B65B 55/06, B65B 55/14

(54) **Sterilisation system and method**

(30) Priority: 12.12.2011 IT PR20110100
(71) Applicant: G.F. S.p.A., 43040 Solignano (Parma) (IT)
(72) Inventor: ABATI, Stefano, 43035 FELINO (PR) (IT)
(74) Representative: Monelli, Alberto

(57) **Abstract**

A system for sterilising pharmaceutical products already packaged in containers, comprising a path (10) for feeding the products packaged in containers, said path (10) comprising:
- a chamber (2) for heating the products already packaged in containers;
- a cooling chamber (3) located downstream of the heating chamber (2); the cooling chamber (3) being separate from the heating chamber (2);
- means (4) for regulating the pressure in the heating chamber (2) and/or in the cooling chamber (3) so as to minimise the risk of the containers being deformed as a result of the pressure variation inside the containers, said pressure variation inside the containers being due to the heating/cooling.

## Description

The present invention relates to a sterilisation system and method. Autoclaves for heating already packaged products, maintaining them at a set temperature and cooling them are well known.

In such cases trays containing already packaged products are introduced into a treatment chamber of the autoclave, in which the processes of heating, maintaining the temperature and cooling take place in succession. At the end of the above-mentioned thermal treatment, by virtue of the period spent at a high temperature, the products are sterilised and the trays are removed from said chamber. At this point a new tray can be positioned inside said chamber and undergo a new treatment cycle.

Though they enable an effective sterilisation action, such autoclaves have some drawbacks. In particular, a first drawback is tied to the relatively small number of products that can be treated in a day. A further drawback is tied to their high energy consumption.

Further drawbacks are tied to the impossibility of introducing autoclaves of this kind in an automated in-line process and the difficulty of ensuring traceability of the products.

Sterilisation systems as described in the patent documents EP1512332, EP0347623, DE2736088, US2010/034938, FR2046017 and GB522957 are also known.

In this context, the technical task at the basis of the present invention is to propose a system and a method for sterilising products already packaged in containers which enables the limits of the prior art to be overcome. In particular, it is an object of the present invention to provide a sterilisation system and method which enables productivity to be increased. A further important object is that of providing a sterilisation system and method which enables energy consumption to be reduced.

The stated technical task and specified objects are substantially achieved by a sterilisation system and method comprising the technical features set forth in one or more of the appended claims.

Additional features and advantages of the present invention will be more apparent from the indicative, and hence non-limiting description of a preferred but non-exclusive embodiment of a sterilisation system and method as illustrated in the appended drawings, in which:
- figure 1 shows a schematic view of a first system according to the present invention;
- figure 2 shows a schematic view of a second system according to the present invention;
- figure 3 shows a schematic view of a third system according to the present invention;
- figure 4 shows a schematic view of a fourth system according to the present invention.

In the appended figures, the reference number 1 indicates a system for sterilising products already packaged in containers. It thus enables a sterilisation both of the products and of the package. The system 1 has broad application, for example, in the packaging of pharmaceutical products. In the case of pharmaceutical products, regulations usually require the sterilisation of already packaged products.

The system 1 comprises a path 10 for feeding the products already packaged in containers. The path 10 is an uninterrupted production line. The path 10 comprises:
- a chamber 2 for heating the products already packaged in containers;
- a cooling chamber 3 located downstream of the heating chamber 2 along said path.

Advantageously the cooling chamber 3 comprises means 30 for at least partially drying the containers containing the products. For example, said at least partial drying means 30 comprise blowers which generate a movement of air inside the cooling chamber 3. In the cooling chamber 3, the products are brought to a temperature close to room temperature (thus avoiding abrupt thermal shocks).

Advantageously, in at least one configuration the heating chamber 2 is sealed.

Advantageously, in at least one configuration the cooling chamber 3 is sealed. The cooling chamber 3 is separate from the heating chamber 2. Advantageously, the system 1 comprises means 4 for regulating the pressure in the heating chamber 2 and/or in the cooling chamber 3 so as to minimise the risk of the containers being deformed as a result of the pressure variation inside the containers, said pressure variation inside the containers being due to the heating/cooling. In fact, the containers are closed and the heating/cooling inside them results respectively in an increase and reduction in pressure.

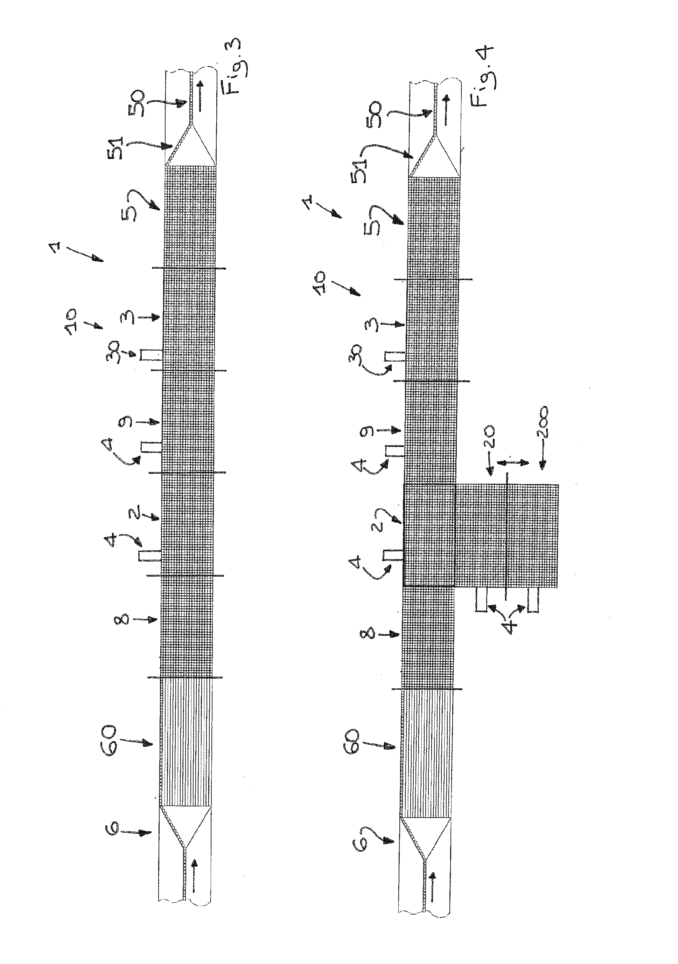

The heating chamber 2 is therefore under counter pressure. Analogously, the cooling chamber 3 is under counter pressure. In the embodiment of figure 4, in addition to the heating chamber 2, the system 1 also comprises at least a first auxiliary heating chamber 20 (in the embodiment of figure 4 there are present a first and a second auxiliary heating chamber 20, 200). The first and/or the second auxiliary heating chambers 20, 200 are sealed and advantageously under counter pressure. The heating chamber 2 and said first auxiliary heating chamber 20 can be moved en bloc (For example, vertically). As better explained below, this feature makes it possible to increase the duration of the heating cycle without penalising the speed of the system. Advantageously, the means 4 for regulating the pressure in the heating chamber 2 can be set independently of the means 4 for regulating the pressure in the cooling chamber 3.

Advantageously, the system 1 comprises a sterilisation chamber 9 in which the already packaged products undergo a thermal treatment. In particular, the products are kept for a predetermined time at a temperature higher than a predetermined temperature (which is thus definable as a "predetermined sterilisation temperature"). The value of this predetermined time and/or of said predetermined sterilisation temperature varies depending on a number of parameters: laws and regulations, types of products, quantities of products, available power, etc. For example, the predetermined sterilisation temperature could range between 100° and 140°C. The predetermined time could be greater than or equal to 10 minutes, for example, it could be greater than or equal to 20 minutes.

Advantageously, the sterilisation chamber 9 can be thermostatically controlled to maintain the desired temperature. The sterilisation chamber 9 thus comprises temperature regulation means. Advantageously, the sterilisation chamber 9 can be thermally insulated. This serves to limit the cooling of the containers due to the heat exchange with the outside environment. Thus the use of additional energy to maintain a constant temperature is limited. Advantageously, the system 1 comprises means for regulating the pressure in the sterilisation chamber 9 (the means for regulating the pressure could for example coincide at least in part with the means for regulating the temperature in the sterilisation chamber 3). For example, the means for regulating the pressure and temperature in the sterilisation chamber 9 could comprise means of supplying hot air. Advantageously, one or more of the other chambers mentioned herein could also be thermostatically controlled and/or insulated.

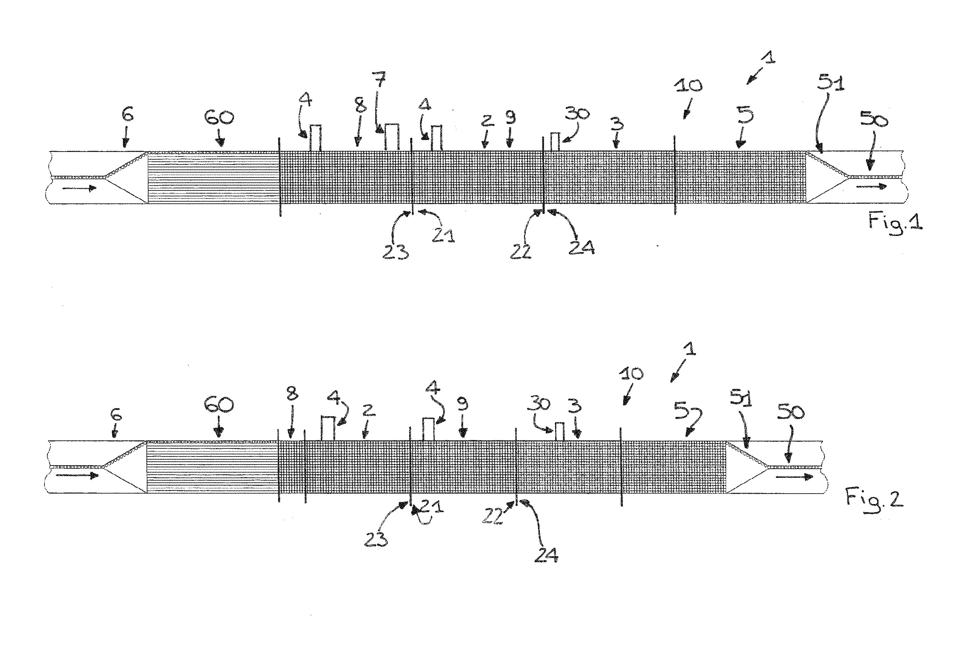

The sterilisation chamber 9 can be:
- coincident with said heating chamber 2 (see for example figure 1); or
- interposed between said heating chamber 2 and said cooling chamber 3 (see for example figures 2-4).

The sterilisation chamber 9 comprises an inlet 21 and an outlet 22 for the products. The inlet 21 is distinct from the outlet 22. In particular, the products pass through the sterilisation chamber 9.

Advantageously (see figure 1), the sterilisation chamber 9 is adjacent to and immediately upstream of the cooling chamber 3. Advantageously, the sterilisation chamber 9 is likewise under counter pressure. There are thus means for regulating the pressure in the sterilisation chamber 9. The means for regulating the pressure in the heating, cooling and sterilisation chambers comprise at least one compressor which introduces air under pressure into the chambers themselves.

Advantageously, the system 1 comprises a first sealed shutter 23 positioned at the inlet of the sterilisation chamber 9.

The system 1 advantageously also comprises a second sealed shutter 24 at the outlet of the sterilisation chamber 9.

The first and second shutters 23, 24 are movable separately from each other.

Advantageously, the cooling chamber 3 likewise comprises an inlet and an outlet for the products; said inlet of the cooling chamber 3 and said outlet of the cooling chamber 3 are distinct from each other.

The cooling chamber 3 could comprise a third sealed shutter positioned at the inlet of said cooling chamber 3. The cooling chamber 3 could comprise a fourth sealed shutter positioned at the outlet of said cooling chamber 3.

In the embodiment of figure 1, the outlet of the sterilisation chamber 9 could also coincide with the inlet of the cooling chamber 3. In this case the second and the third shutter could also coincide.

The system 1 comprises means for automatically transferring (they require no manual intervention) the packaged products from the sterilisation chamber 9 to the cooling chamber 3.

If the sterilisation chamber 9 does not coincide with the heating chamber, the system 1 advantageously also comprises means of transfer from the heating chamber 2 to the sterilisation chamber 9.

The means for transferring the packaged products from the sterilisation chamber 9 to the cooling chamber 3 could comprise a first conveyor belt which extends along the sterilisation chamber 9 and a second conveyor belt which extends along the cooling chamber 3; to allow passage from the first to the second belt it is possible to use a bridge removably positionable at a passage interface. Alternatively, or in addition, an outfeed end of the first belt and an infeed end of the second belt can be moved reciprocally closer/away from each other to allow the passage of the containers from the first to the second belt. The first belt inside the sterilisation chamber 9 could extend along a serpentine or rectilinear path according to specific requirements. The same applies for the second belt inside the cooling chamber 3. Advantageously, the path 10 comprises means 6 for distributing the products packaged in containers into a plurality of rows. The distribution means 6 are upstream of the heating chamber 2 relative to the direction in which the products are fed along the path 10. They typically comprise a dynamic buffer. For example, the distribution means 6 comprise a movable baffle which guides the already packaged products into a plurality of rows side by side.

Advantageously, between the distribution means 6 and the heating chamber 2, the path 10 comprises a buffer 60 for accumulating the already packaged products (in technical language it is a static buffer). Advantageously, but not necessarily, in said accumulation buffer 60 the products are arranged along feed lines set side by side (as distributed by the distribution means 6).

In a particular and non-limiting embodiment each of said side-by-side feed lines comprises an independent conveyor belt, or else the various products arranged side by side along said feed lines rest upon the same conveyor belt. In the latter case the feed lines could be divided by a separator in an elevated position relative to the conveyor belt (for example, guides which define guide lanes for the containers). Advantageously, the system 1 comprises means of transfer from the accumulation buffer 60 to downstream of the cooling chamber 3 (in such a case there could be present a plurality of conveyor belts positioned in succession which could advantageously be moved closer/away at the exchange interface). In a particular embodiment, the means for transferring the packaged products from the sterilisation chamber 9 to the cooling chamber 3 could comprise first means for gripping the containers (typically, the gripping means could act upon a neck of the bottle). For example, the first gripping means could be mobile at least along a length of the sterilisation chamber 9. The means of transfer from the chamber 9 to the chamber 3 could also comprise:
- second container gripping means mobile at least along a length of the cooling chamber 3;
- a star wheel positionable between the first and second container gripping means to allow the passage of the containers from the first to the second gripping means. The means of transfer from the chamber 9 to the chamber 3 could also be present in other areas of the system 1, for example to allow the passage of the containers from the chamber 2 to the chamber 9, from the buffer 60 to the chamber 2, etc.

Advantageously, the heating chamber 2 (see figure 1) comprises means 7 for generating electromagnetic waves for heating the products already packaged in containers. The means 7 for generating electromagnetic waves can comprise radiofrequency generating means (which can be advantageously used in the case of electrolytic solutions such as saline solutions) or else comprise microwave generating means.

Advantageously, in the embodiments of figures 2-4, the sterilisation chamber 9 instead comprises means for spraying a heated fluid. In the embodiment of figure 4, the heating means also comprise means for spraying a heated fluid (for example steam). Downstream of the cooling chamber 3, the path comprises a discharge zone 5. As illustrated by way of example in the appended figures, in the discharge zone 5 the products are aligned in a plurality of rows. Advantageously, the system 1 comprises a line 50 for evacuating the products located downstream of said discharge zone 5. The system 1 further comprises a connector 51, which in succession places the evacuation line 50 in communication with each of said rows of products present in the discharge zone 5. The system 1 comprises means for controlling the distribution means 6 and the connector 51 so as to decide the order in which the containers will exit the system 1 according to the order in which they enter the system 1; in particular, a FIFO (well-known acronym of "first in, first out") logic can be used.

The subject matter of the present invention also relates to a method for sterilising products (preferably pharmaceutical) already packaged in a first group of containers. Advantageously, this method is implemented by a system 1 having one or more of the previously described features. Advantageously, the method follows one or more of the teachings described previously with reference to the system 1.

The method comprises the steps of:
- positioning the first group of containers in a heating chamber 2;
- heating the first group of containers positioned in the heating chamber 2;
- regulating the pressure in the heating chamber 2 so as to minimise the risk of the containers being deformed because of pressure variations inside the first group of containers, said pressure variations inside the first group of containers being due to the heating thereof.

The method further comprises the steps of:
- positioning the first group of containers in the cooling chamber 3, said cooling chamber 3 being distinct from the heating chamber 2;
- cooling the first group of containers (preferably using a "sprinkler" system);
- regulating the pressure inside the cooling chamber 3 so as to counteract the pressure variation inside the first group of containers due to the cooling of the first group of containers.

The step of positioning the first group of containers in the cooling chamber 3 is achieved by means of conveyors, typically belt conveyors. This step is wholly automated.

The method further comprises a step of carrying out a thermal sterilisation treatment on the first group of containers. The step of carrying out the thermal sterilisation treatment includes a sub-step of keeping the first group of containers for at least a predetermined period at a temperature higher than a sterilisation temperature.

The step of keeping the first group of containers for at least a predetermined period at a temperature higher than a sterilisation temperature follows the heating step and takes place:
- in said heating chamber 2, which thus coincides with a sterilisation chamber 9; or
- in a sterilisation chamber 9 which, along a feed path of the first group of containers, is interposed between the heating chamber 2 and the cooling chamber 3.

The step of positioning the first group of containers in the cooling chamber 3 is preceded by the steps of:
- evacuating a group of containers distinct from the first group from the cooling chamber 3;
- increasing the pressure in the cooling chamber 3 so as to bring it close to that of the sterilisation chamber 9. At this point the first group of containers can be transferred from the sterilisation chamber 9 to the cooling chamber 3. The step of positioning the first group of containers in a heating chamber 2 comprises the step of transferring the first group of containers from an accumulation zone 8 to the heating chamber 2; the step of heating the first group of containers in the heating chamber 2 is at least partly simultaneous with a step of stacking a group of containers distinct from the first group in the accumulation zone 8.

The step of positioning the first group of products in the cooling chamber 3 is followed by the steps of:
- separating the sterilisation chamber 9 and the cooling chamber 3 by means of at least one movable partition;
- introducing a group of containers distinct from the first group into the sterilisation chamber 9 (possibly after having reduced the pressure in the sterilisation chamber 9 -as in the case of figure 1-).

In the case of figures 2-4, the method comprises the step of positioning the first group of containers in said sterilisation chamber 9 located downstream of the heating chamber 2 along the feed direction of the first group.

In the case of figures 2 and 3, the step of positioning the first group of containers in the heating chamber 9 involves transferring said first group from the heating chamber 2 to the sterilisation chamber 9 (this step is preceded by the opening of a partition which places the heating chamber 2 and the sterilisation chamber 9 in fluid communication; after the first group of containers has been positioned in the sterilisation chamber 9 the partition is closed and the thermal treatment is carried out). In the case of figure 4, the system 1 comprises a first auxiliary heating chamber 20 which moves as one with the heating chamber 2. The step of positioning the first group of containers in the heating chamber 2 is followed by a first procedure which includes a step of moving the heating chamber 2 from a first to a second position; in the second position the first procedure envisages positioning a second group of containers in the auxiliary heating chamber 20; in the first position the heating chamber 2 is aligned with the accumulation zone 8 (the inlet of the chamber 2 is facing the zone 8). In the second position the auxiliary heating chamber 20 is aligned with the accumulation zone 8 (the inlet of the chamber 20 is facing the zone 8). The first procedure provides for the first group of containers to be heated in the heating chamber 2 at least during the introduction of the second group into the first auxiliary heating chamber 20. The first procedure advantageously provides for the heating chamber 2 to be then repositioned in the first position so as to allow the first group to exit from the heating chamber 2 toward the cooling chamber 3 (passing through the sterilisation chamber 9).

Analogously, the first procedure provides for the second group of containers to be heated in the first auxiliary heating chamber 20 also after the first group of containers has been discharged from the heating chamber 2. The first procedure likewise provides for the second group of containers to be transferred from the first auxiliary chamber 20 to the cooling chamber 3. The second group of containers is heated in the first auxiliary heating chamber 20 for the same amount of time as the first group of containers in the heating chamber 2. In practice, the heating in the heating chamber 2 and in the first auxiliary heating chamber 20 also takes place during the movement from the first to the second position and vice-versa.

Advantageously, there could also be present a second auxiliary heating chamber 200 which is solidly joined with the heating chamber 2 and the first auxiliary heating chamber 20. The second auxiliary heating chamber 200 is intended to receive a third group of containers when the heating chamber 2 takes on a third position; the third position of the heating chamber 2 corresponds to an alignment of the accumulation zone 8 and second auxiliary heating chamber 200 (and it is therefore possible for the containers to be transferred from the accumulation zone 8 to the chamber 200). In such a case, the first and second groups of containers are heated also during the introduction of the third group into the second auxiliary heating chamber 200. Advantageously, the system 1 comprises means for regulating the pressure in the first and/or in the second auxiliary heating chambers 20, 200 to minimise the risk of the containers being deformed because of the pressure variation therein, ascribable to the heating.

With reference to figure 1, the sterilisation method comprises one or more of the following steps:
i) stacking the first group of containers in the accumulation zone 8 (this step is at least partly simultaneous with the carrying out of a thermal treatment on other containers positioned in the heating chamber 2 and in the cooling chamber 3);
ii) opening a sealed inlet partition of the heating chamber 2, positioning the first group of containers in the heating chamber 2, and closing the sealed inlet partition of the heating chamber 2;
iii) heating the first group of containers positioned in the heating chamber 2 and increasing the pressure in the heating chamber 2;
iv) carrying out a thermal treatment of keeping the first group positioned in the heating chamber 2 (which now acts as a sterilisation chamber 9) at a high temperature for at least a predetermined period;
v) opening a sealed outlet partition and transferring into the cooling chamber 3; the pressure in said cooling chamber 3 having been previously increased to place it in equilibrium with that of the heating chamber 2;
vi) closing the sealed outlet partition of the heating chamber 2 and carrying out a cooling cycle (for example by following a predetermined temperature-time profile); the cooling cycle is accompanied by a reduction in the pressure inside the cooling chamber 3;
vii) carrying out at least a partial drying of the first group of containers positioned in the cooling chamber 3;
viii) positioning the first group of containers in a discharge zone 5. Advantageously, the first group remains in the heating chamber 2 for substantially the same length of time as in the cooling chamber 3.

In the case of figures 2, 3, 4, the method follows that of figure 1 with a few variants; in fact, the heating of the first group of containers and the carrying out of a thermal treatment take place in two separate sealed chambers; in particular, the step of keeping the first group at a predetermined temperature takes place in a sterilisation chamber 9 downstream of the heating chamber 2 (typically, the heating chamber 9 is adjacent to the heating chamber 2).

In the case of figure 2, moreover, the heating of the first group takes place for a shorter duration compared to the time it remains in the sterilisation chamber 9. The accumulation zone 8 upstream of the heating chamber 2 comprises a buffer which allows an accumulation of containers for an amount of time that is substantially equal to the duration of the heating of the first group.

In the case of figure 3, the sum of the times for which the first group remains in the heating chamber 2 (for example 20 minutes) and in the sterilisation chamber 9 (for example 20 minutes) is greater than the amount of time it remains in the single heating/sterilisation chamber 2 of figure 1. This makes it possible to reduce the installed power of the system 1 and enables a slower heating of the containers.

In the case of figure 4, upstream and downstream of the heating chamber 2 the method follows that of figure 3, but the first procedure described previously is also implemented.

The invention thus conceived enables many advantages to be achieved.

First of all, it makes it possible to speed up the production line, since the main operations are carried out in series in distinct chambers. Secondly, it enables a reduction in costs, since every chamber is maintained under steady state conditions for a longer period of time, thus minimising wasteful temperature and pressure variations.

## Claims

1. A system for sterilising pharmaceutical products already packaged in containers, comprising a path (10) for feeding the products packaged in containers, said path (10) comprising:
- a chamber (2) for heating the products already packaged in containers;
- a cooling chamber (3) located downstream of the heating chamber (2); the cooling chamber (3) being separate from the heating chamber(2);
- means (4) for regulating the pressure in the heating chamber (2) and/or in the cooling chamber (3) so as to minimise the risk of the containers being deformed due to the pressure variation inside the containers, said pressure variation inside the containers being ascribable to the heating/cooling of the containers;
- a sterilisation chamber (9) in which the already packaged products are kept for a predetermined time at a temperature higher than a predetermined sterilisation temperature, said sterilisation chamber (9) being:
i) coincident with said heating chamber (2) or
ii) interposed between said heating chamber (2) and said cooling chamber (3) along said path (10);
said path (10) being **characterised in that** it comprises:
- means (6) for distributing the products packaged in containers into a plurality of rows, said distribution means (6) being upstream of said heating chamber (2) relative to the direction in which the products are fed along said path;
- a discharge zone (5) located downstream of the cooling chamber (3), the products in the discharge zone (5) being aligned in a plurality of rows;
- a line (50) for evacuating the products located downstream of said discharge zone (5); the system (1) comprising a connector (51), which in succession places the evacuation line (50) in communication with each of said rows of products present in the discharge zone (5); the system (1) comprising means for controlling the distribution means (6) and the connector (51) so as to decide the order in which the containers will exit the system (1) according to the order in which they enter the system (1) using a FIFO logic.

2. The system according to claim 1, **characterised in that** the sterilisation chamber (9) comprises an inlet (21) and an outlet (22) for the products, the inlet (21) being distinct from the outlet (22).

3. The system according to claim 2, **characterised in that** it comprises:
- a first sealed shutter (23) positioned at the inlet (21) of the sterilisation chamber (9);
- a second sealed shutter (24) positioned at the outlet (22) of the sterilisation chamber (9);
the first and the second shutter (23, 24) being movable separately from each other.

4. The system according to claim 1 or 2 or 3, **characterised in that** it comprises means for transferring the packaged products from the sterilisation chamber (9) to the cooling chamber (3).

5. The system according to any of the preceding claims, **characterised in that** said heating chamber (2) comprises means (7) for generating electromagnetic waves for heating the products already packaged in containers.

6. A method for sterilising pharmaceutical products packaged in a first group of containers using a system according to claim 1 and comprising the steps of:
- positioning the first group of containers in the heating chamber (2);
- heating the first group of containers positioned in the heating chamber (2);
- regulating the pressure in the heating chamber (2) so as to minimise the risk of the containers being deformed due to the pressure variation inside the first group of containers, said pressure variation inside the first group of containers being ascribable to the heating thereof;
- positioning the first group of containers in the cooling chamber (3), said cooling chamber (3) being distinct from the heating chamber (2);
- cooling the first group of containers;
- regulating the pressure in the cooling chamber (3) so as to minimise the risk of the containers being deformed due to the pressure variation inside the first group of containers, said pressure variation inside the first group of containers being ascribable to the cooling of the first group of containers;
- keeping the first group of containers for at least a predetermined period at a temperature higher than a sterilisation temperature; said step of keeping the first group of containers for at least a predetermined period at a temperature higher than the sterilisation temperature following the step of heating the first group of containers and taking place:
i) in said heating chamber (2), which thus coincides with a sterilisation chamber (9); or
ii) in a sterilisation chamber (9) which, along a feed path (10) of the first group of containers, is interposed between the heating chamber (2) and the cooling chamber (3);
- distributing the containers of the first group in a plurality of rows; said step taking place before the step of heating the first group of containers;
- accumulating the first group of containers in the discharge zone (5) located downstream of the cooling chamber (3); the containers in said discharge zone (5) being aligned in a plurality of rows;
- evacuating the first group of containers along the evacuation line (50) located downstream of the discharge zone (5) by means of the connector (51), which in succession places the evacuation line (50) in communication with each of the rows of containers present in the discharge zone (5); the step of evacuating the first group of containers including discharging the containers from said system according to the order of entry into the system following a FIFO logic.

7. The method according to claim 6, **characterised in that** the step of positioning the first group of containers in the cooling chamber (3) is preceded by the steps of:
- evacuating a group of containers distinct from the first group from the cooling chamber (3);
- increasing the pressure in the cooling chamber (3) so as to bring it close to that of the sterilisation chamber (9).

8. The method according to claim 6 or 7, **characterised in that** the step of positioning the first group of products in the cooling chamber (3) is followed by the steps of:
- separating the sterilisation chamber (9) and the cooling chamber (3) by means of at least one movable partition;
- introducing a group of containers distinct from the first group into the sterilisation chamber (9).

9. The method according to claim 6 or 7 or 8, **characterised in that** the step of positioning the first group of containers in the heating chamber (2) comprises the step of transferring the first group of containers from an accumulation zone (8) to the heating chamber (2); the step of heating the first group of containers being at least partly simultaneous with a step of stacking a group of containers distinct from the first group in the accumulation zone (8).

10. The method according to claim 9, **characterised in that** the step of positioning the first group of containers in the heating chamber (2) is followed by a first procedure comprising the following steps:
- moving the heating chamber (2) from a first to a second position, a first auxiliary heating chamber (20) moving as one with the heating chamber (2), in the first position an inlet of the heating chamber (2) facing the accumulation zone (8), in the second position an inlet of the first auxiliary heating chamber (20) facing the accumulation zone (8); the step of heating the first group of containers in the heating chamber (2) taking place at least in part during the introduction of the second group of containers into the first auxiliary heating chamber (20);
- repositioning the heating chamber (2) in the first position to allow the discharge of the first group from the heating chamber (2) and the transfer thereof to the cooling chamber (3); the second group of containers being heated in the first auxiliary heating chamber (20) also after the discharge of the first group of containers from the heating chamber (2);
- discharging the second group of containers from the first auxiliary heating chamber (20) and transferring it to the cooling chamber (3).
